(19) Europäisches Patentamt
European Patent Office
Office européen des brevets

(11) **EP 2 412 798 A1**

(12) **EUROPEAN PATENT APPLICATION**

(43) Date of publication:
**01.02.2012 Bulletin 2012/05**

(51) Int Cl.:
***C12N 5/00*** (2006.01)

(21) Application number: **10170889.9**

(22) Date of filing: **27.07.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Gambro Lundia AB**
**220 10 Lund (SE)**

(72) Inventors:
• **Neubauer, Markus**
**82377 Penzberg (DE)**
• **Kieferle, Verena**
**72072 Tübingen (DE)**

(74) Representative: **Hornung, Veronika Margot**
**c/o Gambro Dialysatoren GmbH**
**Holger-Crafoord-Strasse 26**
**72379 Hechingen (DE)**

(54) **Biomimetic membrane for cell expansion**

(57) The invention relates to a membrane which can be used for cultivating adherent or suspension cells, in particular adherent cells, wherein said membrane allows for the adhesion and proliferation of the cells due to modification of the membrane surface with a combination of at least one extracellular matrix protein, at least one extracellular matrix (proteo-) glycan, and at least one heparin-binding growth factor. The invention further relates to a method for preparing said modified or coated membrane which can be used for the cultivation of cells, in particular adherent cells, and to methods of using such membrane for the cultivation of cells, in particular adherent cells.

Figure 2

**Description**

**Technical Field**

The invention relates to a membrane which can be used for cultivating adherent or suspension cells, in particular adherent cells, wherein said membrane allows for the adhesion and proliferation of the cells due to modification of the membrane surface with a combination of at least one extracellular matrix protein, at least one extracellular matrix (proteo-) glycan, and at least one heparin-binding growth factor. The invention further relates to a method for preparing said modified or coated membrane which can be used for the cultivation of cells, in particular adherent cells, and to methods of using such a membrane for the cultivation of cells, in particular adherent cells.

**Background of the Invention**

The aim of the current invention was the identification of membranes which exhibit growth characteristics substantially similar to tissue culture polystyrene (TCPS) plates which represent today's gold standard for cell expansion using culture flasks or cell stacks. Principal characteristics to be measured were cell expansion rate, attachment efficiency of cells onto membranes, and characteristics of cell post-expansion including visual morphology control and phenotype control by flow cytometry. The biomimetic membranes of the invention can be used equally efficient in various geometries, such as flat sheet or hollow fiber membranes.

The invention particularly relates to membranes which can, for example, be used for culturing, growing, storing and/or expanding adherent cells of various types. In the context of the present invention, the expression "cell culture" or "culturing (of) cells" shall comprise all such uses, i.e. the adherence, maintenance, growth, expansion, differentiation, molecular biological modification (e.g. transfection), and/or storage of cells of different types.

Like most cells in vivo, many cells are adherent cells, or anchorage-dependent cells; that is, they can metabolize and divide only if they are attached to a surface or substrate. Only cells of the circulatory system (e.g. lymphocytes and red blood cells) and other cell types such as hematopoietic stem cells, hepatocytes, CHO cells, etc. grow unattached and suspended in solution *in vitro*. While many anchorage-dependent cells may grow on glass or synthetic surfaces, these cells often lose their ability to differentiate and respond to hormones. The loss of cellular morphology not only entails a loss of function, but also prevents regenerative power in a longer-term culture system. Longer-term cultivation would however be of great significance, for example, with the use of human cells for tissue culture, and many cells are not available in any quantity. For this reason, such tissue culture dishes are often coated with extracellular matrix components such as collagen or fibronectin. The use of xenogenic factors may be seen critically, especially if the cells as such or on a matrix as used for medical treatment of human beings, as it will bring along risks of contamination and may result in adverse reactions in the patient treated. However, it may be necessary to provide means for the highly efficient culturing of cells where for example, only a minimum of cells will form the starting point for a cell culture. It is therefore one object of the present invention to provide membranes which will be highly efficient for the culturing of adherent cells and which will outperform even today's current gold standard with regard to attachment efficiency and/or cell expansion rate.

The failure of cells to grow on certain surfaces or keep their abilities is, for example, a major limitation of current tissue culture techniques. Tissue cultures are a potential source of tissues and organs which could be used for transplantation into humans. For example, tissue cultured skin cells could potentially be used in skin grafts. The aim is to develop biological substitutes that can restore and maintain normal function, for example, by the use of acellular matrices, which will depend on the body's ability to regenerate for proper orientation and direction of new tissue growth, or by the use of matrices or membranes with cells adhered thereto (Atala (2006): Recent developments in tissue engineering and regenerative medicine. Curr. Opin. Pediatr. 16, 167-171). Cells can also be used for therapy via injection, either with carriers or alone. In such cases, the cells need to be expanded in culture, attached to a support matrix, and then reimplanted into the host after expansion. Veterinary therapeutic applications are available today and may represent an additional application of membranes for cell cultivation.

The ability to culture cells, especially adherent cells, is important also because they represent biological "factories" capable of producing large quantities of bio products such as growth factors, antibodies and viruses. These products can then be isolated from the cell cultures and used, for example, to treat human diseases.

Additionally, cell cultures are emerging tools for biocompatibility and toxicology studies in the field of pharmaceutical and life science industry.

Finally, tissue cultures usually comprise cells from only one or a few tissues or organs. Consequently, cell cultures provide scientists a system for studying the properties of individual cell types without the complications of working with the entire organism.

A known method for culturing adherent cells involves a hollow fiber membrane bioreactor. In this system, the cells are generally attached to the lumen of a cylindrical hollow fiber membrane. Culture media and oxygen flows through the center of the cylindrical hollow fiber membrane. The molecular weight cut-off of the membrane permits nutrients and oxygen to reach the cells without allowing the cells to escape.

A variety of polymers has been suggested for producing semi-permeable membranes for cell and tissue culture (US 2007/269489). They include polyalginate, polyvinylchloride, polyvinylidene fluoride, polyurethane isocyanate, cellulose acetate, cellulose diacetate, cellulose triacetate, cellulose nitrate, polysulfone, polyethersulfone, polystyrene, polyurethane, polyvinyl alcohol, polyacrylonitrile, polyamide, polymethylmethacrylate, polytetrafluoroethylene, polyethylene oxide and combinations of such polymers. The polymeric support may also consist of polyethylene terephthalate (PET) or polycarbonate. Further materials which were suggested, for example, as scaffolds for transplantable tissue material, are cellulose or macroporous collagen carriers, or biodegradable matrices.

It is of course also known to perform the culturing of cells on flat sheet membranes or flat surfaces.

Apart from the problem of identifying membrane compositions which could be used as a matrix for the cultivation of adherent cells, membranes currently known in the art are able to promote and sustain adherence, expansion, differentiation and extended life-span with some kind of pre-treatment of said membranes or matrices, including the addition of exogenous factors, such as, for example, fibronectin, laminin or collagen.

Many references describe the use of, for example, either fibronectin, growth factors such as FGF-2 or heparin for preparing matrices which can be used for cell attachment and cell cultivation. However, no reference could be identified which teaches the modification of a membrane surface with a combination comprising at least one extracellular matrix protein, at least one extracellular matrix (proteo-) glycan, and at least one heparin-binding growth factor, such as, for example, the combination of fibronectin, a growth factor and heparin.

For example, Fissell (2006) in Expert Rev. Med. Devices 3(2), 155, reviews efforts with regard to developing an artificial kidney based on adhering renal tubule cells to a synthetic polysulfone-based hollow-fiber membrane. In this case the membrane is coated with ProNectin-L™ in order to promote attachment of the cells.

US-A 6,150,164 and US-A 6,942,879 both present elaborate ways towards a bioartificial kidney based on renal cells such as, for example, endothelial cells or so-called renal stem cells, which are seeded into hollow fibers. Hollow fiber membranes which are mentioned as being useful are based on cellulose, polyacrylonitrile, polysulfone and other components or copolymers thereof. The internal and external surface of the hollow fiber is pre-coated with suitable extracellular matrix components (EMC) including Type I collagen, Type IV collagen, laminin, Matrigel, proteoglycan, fibronectin and combinations thereof. Only after such treatment the cells can be seeded.

US 2006/213836 A1 describes the membranes which comprise at least one surface treatment or modification for promoting the attachment of specific animal cells to the membrane, promotes attachment of desirable proteins, inhibits undesirable protein deposition on the membrane, or inhibits blood coagulation on or in the vicinity of the membrane. Such modifications may include un-patterned adsorption or covalent linkage to the membrane surface of RGD peptide moieties, integrins, fibronectin, heparin, laminin, collagens, or polyethylene glycol moieties. Also described is the promotion of the attachment of cells such as endothelial or epithelial cells. The use of a combination of at least one extracellular matrix protein, at least one extracellular matrix (proteo-) glycan, and at least one heparin-binding growth factor, such as, for example, a specific combination of fibronectin, FGF-2 and heparin is not described.

US 2003/138950 A1 provides methods and composition to build living tissue covered stents and the like, wherein the surfaces (e.g., the lumen-wall contacting surface and/or the lumen exposed surface) are coated with extracellular matrix material such as, for example, fibronectin, fibrin or collagen prior to contact with a tissue sheet in order to promote adherence of the tissue sheet to the stent. The use of a specific combination of a combination of at least one extracellular matrix protein, at least one extracellular matrix (proteo-) glycan, and at least one heparin-binding growth factor is not described.

US 2007/003916 A1 discloses synthetic anatomical models, which are designed to enable simulated use testing by medical device, pharmaceutial, and consumer product developers. The attachment of cells to the substrate is enhanced by coating the substrate with compounds such as basement membrane components, agar, agarose, gelatin, gum arabic, collagens types I, II, III, IV, and V, fibronectin, FGF, laminin, glycosaminoglycans and mixtures thereof. However, heparin has not been considered alone or in combination with one or more of the above mentioned factors.

Ishihara et al. (2000): "Heparin-carrying polystyrene to mediate cellular attachment and growth via interaction with growth factors", Journal of Biomedical Materials Research 50 (2), 144-152 describe that heparin-carrying PS (HCPS) is especially able to retain the binding of heparin-binding growth factors (GFs) such as vascular endothelial GF 165 (VEGF165) or fibroblast GF 2 (FGF-2). Human skin fibroblast cells, human coronary smooth muscle cells, and human coronary endothelial cells have good adherence to such HCPS-coated plate. However, the use of a specific combination of at least one extracellular matrix protein, at least one extracellular matrix (proteo-) glycan, and at least one heparin-binding growth factor is not described.

US 6,921,811 B2 describes coatings and coated contacting surfaces of medical devices, wherein the coating includes silyl-heparin with one or more bioactive molecules bound to the heparin. Said bioactive molecules include adhesive molecules, such as fibronectin for promoting cellular attachment, growth factor molecules, such as basic fibroblast growth factor for promoting cellular growth, and a variety of other therapeutic molecules for effecting one or more therapeutic purposes. However, the reference fails to disclose the use of a combination of fibronectin, FGF-2 and heparin together.

## Summary of the Invention

In the present invention, membranes are disclosed which are treated, after preparation, with a combination of at least one extracellular matrix protein, at least one extracellular matrix (proteo-) glycan, and at least one heparin-binding growth factor, such as, for example, FGF-2, heparin and fibronectin. Such modified membrane proved to be surprisingly improved with regard to the cultivation of cells than those membranes or TCPS know in the art. The present invention is also directed to a method of preparing such membrane. The present invention is also directed to methods of using the membrane for promoting cell attachment and for the cultivation of cells, in particular adherent cells, especially mesenchymal stem cells (MSC). Preferred membranes in the context of the present invention are polysulfone-based, polyethersulfone-based or poly(aryl)ethersulfone-based synthetic membranes, comprising, in addition, PVP and optionally low amounts of further polymers, such as, for example, polyamide or polyurethane.

## Brief Description of the Drawings

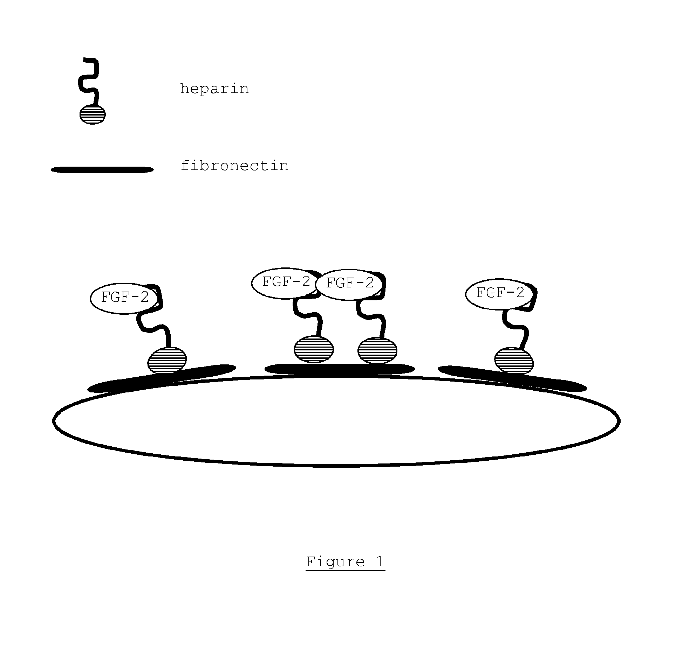

Figure 1 shows the schematic biomimetic membrane setup. The initially formed complex comprising, for example, fibronectin (FN), heparin (Hep) and FGF-2 is bound to the matrix surface via fibronectin. Heparin and FGF-2 are bound to fibronectin, thus forming a triple complex attached to the membrane matrix. For abbreviations used see also Table I.

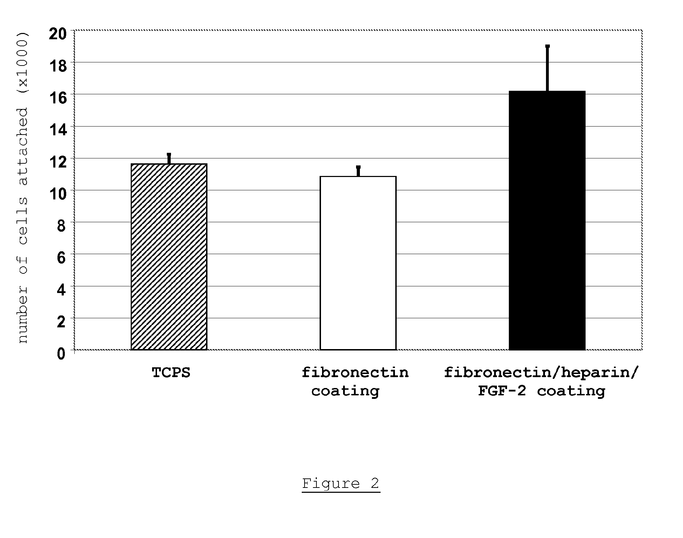

Figure 2 shows the number of MSC grown from unprocessed bone marrow on a poly(aryl)ethersulfone-based membrane treated with a complex comprising fibronectin, heparin and FGF-2 (black column) relative to the number of MSC grown on the same membrane which was treated only with fibronectin (white column) and on standard TCPS (grey column) after 1 day. For abbreviations used see Table I.

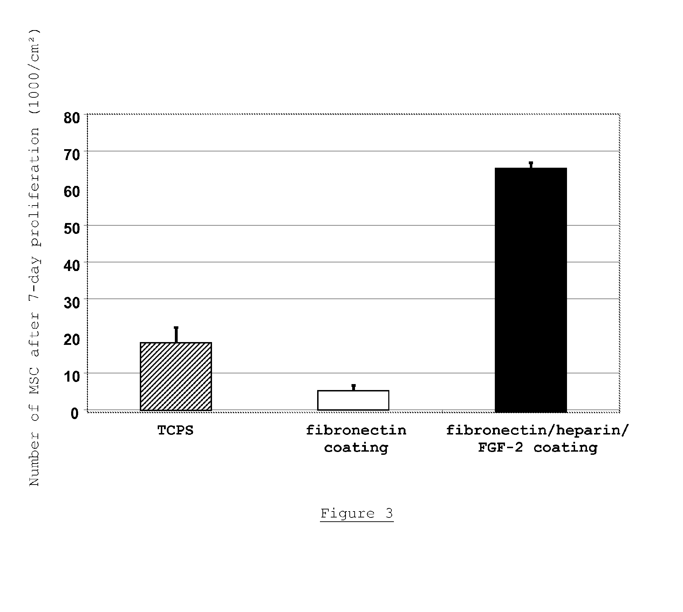

Figure 3 shows the number of MSC after seven days of proliferation ($1000/cm^2$) on a poly(aryl)ethersulfone-based membrane (U9000® from Gambro Lundia AB) treated with a complex comprising fibronectin, heparin and FGF-2 (black column) relative to the number of MSC which attached to the same membrane which was treated only with fibronectin (white column) and to standard TCPS (grey column). For abbreviations used see Table I.

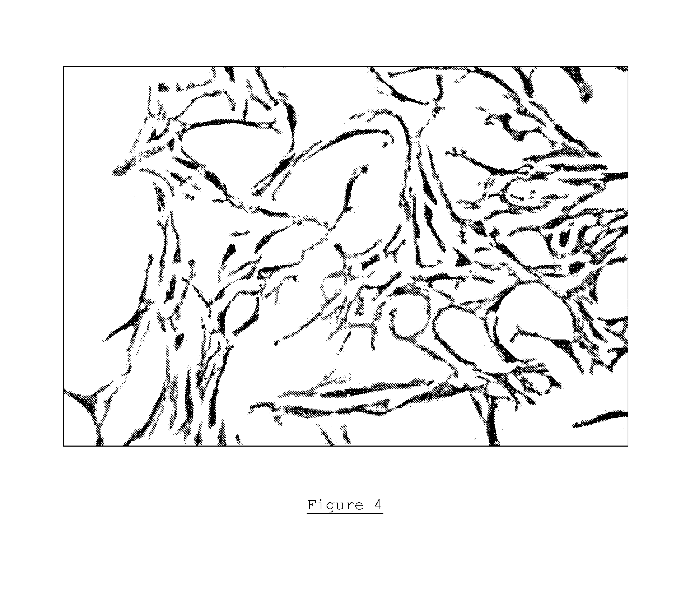

Figure 4 shows the morphology of re-plated MSC which were expanded on conventional TCPS dishes (control). MSC showed the typical spindle-shaped morphology when re-plated on TCPS.

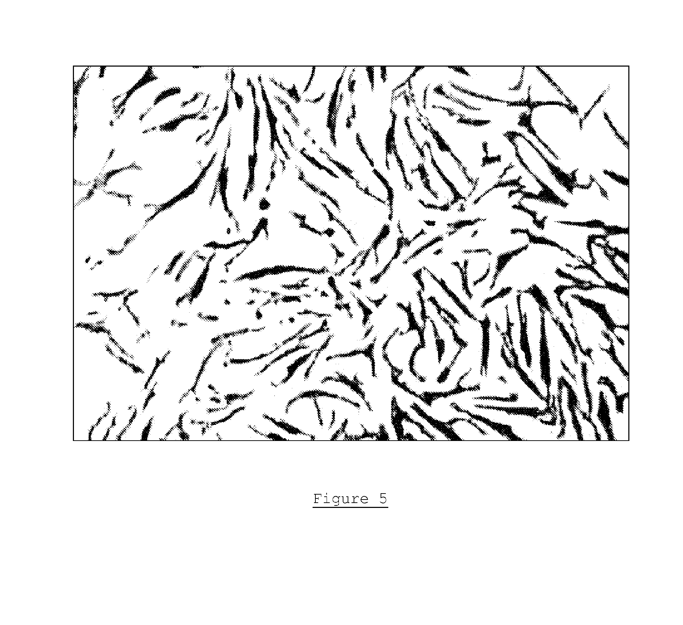

Figure 5 shows the morphology of re-plated MSC which were expanded on a poly(aryl)ethersulfone-based membrane (U9000® from Gambro Lundia AB) treated with fibronectin. MSC showed the typical spindle-shaped morphology.

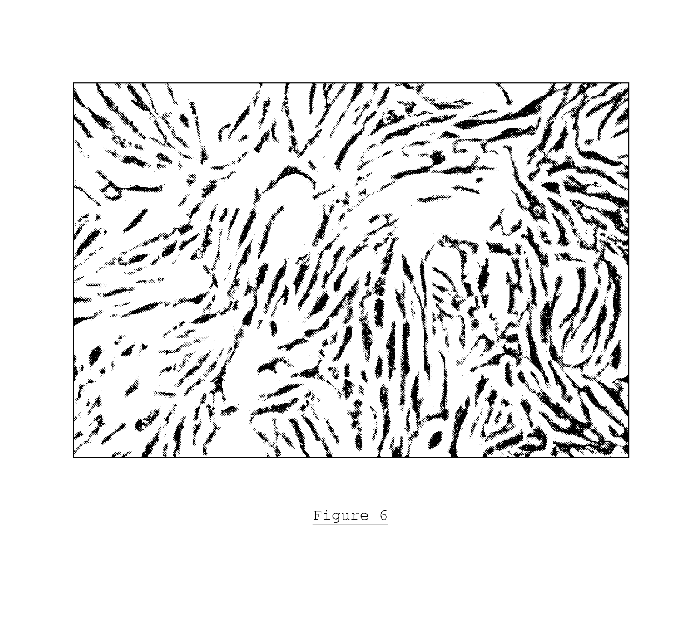

Figure 6 shows the morphology of re-plated MSC which were expanded on a membrane treated with a complex comprising fibronectin, heparin and FGF-2 according to the invention. The MSC showed the typical spindle-shaped morphology.

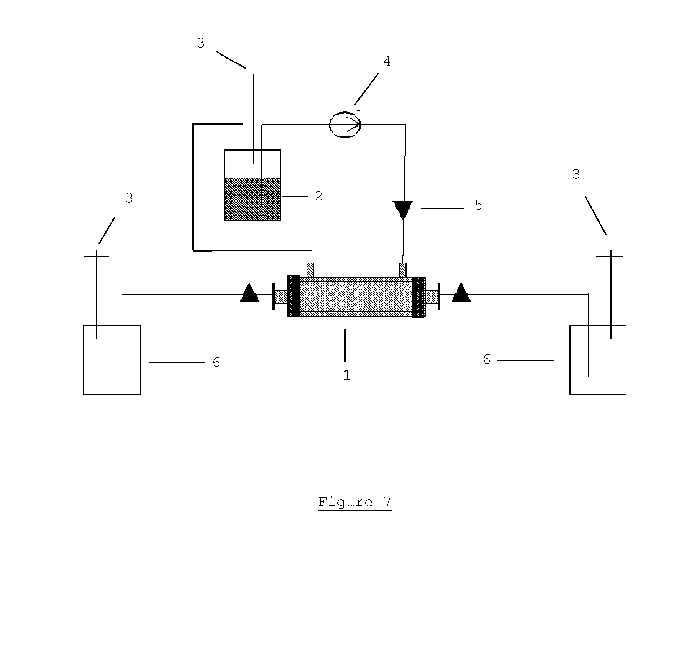

Figure 7 depicts a possible setup for a cell culturing device comprising a hollow fiber membrane bundle according to the invention. The system shown has been designed to allow for the medium to flow and be exchanged on the extracapillary side. The membrane is part of a filter (1) which is connected to culture medium (2) which can be added to the reservoir through a sterile filter (3). The medium is pumped (4) to the filter comprising the hollow fiber membrane of the invention. Samples can be taken at (5). The necessary gas is provided by letting the medium drip into the medium reservoir (2) which is in contact with the surrounding air.

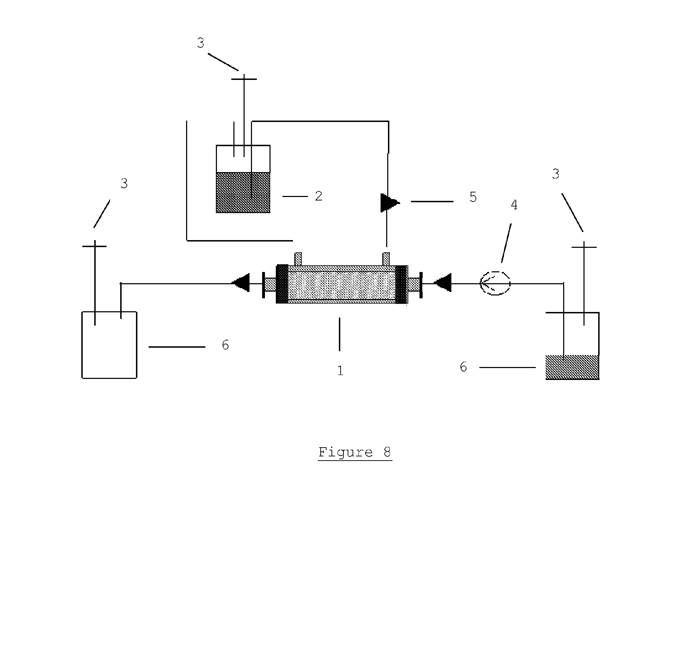

Figure 8 depicts a possible setup for a cell culturing device comprising a hollow fiber membrane bundle according to the invention. The system shown has been designed to allow for the medium to flow and be exchanged on the intracapillary side. The membrane is part of a filter (1) which is connected to culture medium (6) which can be added to the reservoir through a sterile filter (3). The medium is pumped (4) to the filter comprising the hollow fiber membrane of the invention. Samples can be taken at (5). The necessary gas is provided by letting the medium drip into the medium reservoir (6) which is in contact with the surrounding air.

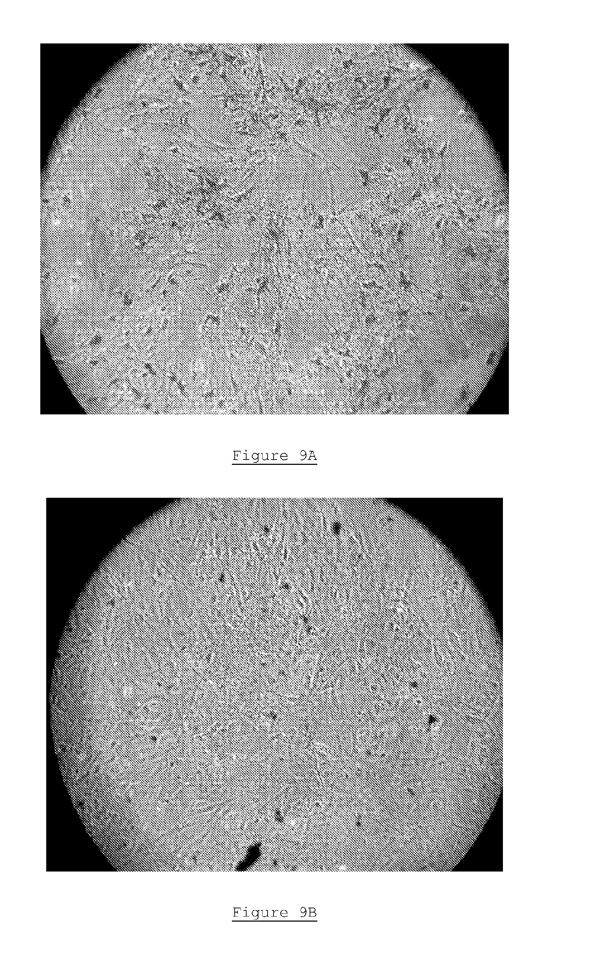

Figure 9 shows the differentiation of cells which were grown on membranes according to the invention and as described in Example 4. Figure 9A shows that the MSC have differentiated into osteogenic cells, indicating that the stem cell potential was maintained. The dark spots in Figure 9A represent the mineralized matrix which is typical for osteogenesis. Figure 9B depicts MSC which have differentiated into adipogenic cells. Here, the dark spots show stained lipid droplets which prove that the cells underwent adipogenic differentiation.

## Detailed Description of the Invention

It was an object of the present invention to devise a membrane which has improved characteristics with regard to the cultivation of cells, especially adherent cells, based on a modification of a membrane with a complex comprising at least one defined extracellular matrix protein to which an extracellular matrix (proteo-) glycan is bound, wherein the glycan has the capacity to bind a growth factor, and a heparin-binding growth factor. Such improvement comprises, for example, increasing the number of cells which will attach to the surface of the matrix after seeding and/or the number of cells which will be harvested from the cultivation matrix after a defined proliferation period.

In one embodiment of the invention the extracellular matrix protein is chosen from the group comprising fibronectin, collagen, pronectin F and laminin, collectively referred to as "glycoproteins" in the context of the present invention.

In a preferred embodiment of the invention, the extracellular matrix protein is fibronectin.

In yet another embodiment of the invention the extracellular matrix (proteo-) glycan is a glycosaminoglycan, chosen from the group comprising, for example, heparin, heparan sulfate, hyaluronan, dermatan, keratan and chondroitin sulfate. Members of the glycosaminoglycan family vary in the type of hexosamine, hexose or hexuronic acid unit they contain (e.g. glucuronic acid, iduronic acid, galactose, galactosamine, glucosamine). They also vary in the geometry of the glycosidic linkage. Any such variations are deemed to be aspects of the present invention.

In yet another preferred embodiment of the present invention, the glycosaminoglycan is heparin or heparan sulfate, especially heparin.

In yet another embodiment of the invention the growth factor is a FGF-2, PDGF or EGF. In another particular embodiment the growth factor is FGF-2.

In another particularly preferred embodiment of the invention, fibronectin is used for modifying the substrate membrane together with FGF-2 and heparin.

It is, of course, possible to use different extracellular matrix proteins on a given membrane. For example, it is possible to use different proteins such as fibronectin and laminin together. On the other hand, if fibronectin is ti be used, the fibronectin may be derived from different sources or may consist of different variants of fibronectin comprising, for example, different types, fragments or domains of the protein.

It is also possible, in combination with fibronectin, to use different glycosaminoglycans such as, for example, different glycosaminoglycans such as heparin and heparin sulfate together. On the other hand, it is possible to use, for example, different heparin variants. For example, the heparin used may be derived from different sources or may consist of different variants of heparin, comprising, for example, varying types, domains or fragments of the protein.

It is also possible, in combination with fibronectin and heparin, to use different growth factors such as, for example, FGF-2 in combination with PDGF and/or EGF. It is also possible to use a growth factor such as FGF-2 wherein the growth factor may be derived from different sources or may consist of different variants, comprising, for example, varying types, domains or fragments of the protein.

Fibronectin is a high-molecular weight (~440kD) extracellular matrix glycoprotein that binds to membrane-spanning receptor proteins called integrins. In addition to integrins, fibronectin also binds extracellular matrix components such as collagen, fibrin and heparan sulfate proteoglycans. There are four fibronectin-binding domains, allowing fibronectin to associate with other fibronectin molecules. One of these fibronectin-binding domains is referred to as the "assembly domain", and it is required for the initiation of fibronectin matrix assembly. Importantly, there is also a "cell-binding domain" of fibronectin which covers the RGD sequence (Arg-Gly-Asp) and is the site of cell attachment on the cell surface. The "synergy site" has a role in modulating fibronectin's accociation with integrins.[5] Fibronectin also contains domains for fibrin-binding, collagen-binding, fibulin-1-binding, heparin-binding and syn-decan-binding. Fibronectin is, among other functions, involved in cell adhesion, growth, migration and differentiation. As such, cellular fibronectin is assembled into the extracellular matrix, an insoluble network that separates and supports the organs and tissues of an organism. Fibronectin as used for preparing a membrane according to the invention can be purchased, for example, from Chemicon.

Native heparin is a polymer with a molecular weight ranging from 3 kD to 30 kD, although the average molecular weight of most commercial heparin preparations is in the range of 12 kD to 15 kD. Heparin is a member of the glycosaminoglycan family of carbohydrates (which includes the closely-related molecule heparan sulfate) and consists of a varia-bly-sulfated repeating disaccharide units. Heparin as used for preparing a membrane according to the invention can be purchased, for example, from ratiopharm.

Growth factors are substances that stimulate the growth of cells. In particular in cell biology, growth factors are proteins that bind to receptors on the target cell surface with the primary result of modulating cellular proliferation and/or differentiation and therefore, growth factors direct the action of specific genes in the targeted cell. Many growth factors are quite versatile, stimulating cellular division in numerous different cell types, while others are specific to a particular cell-type. Epidermal growth factors (EGF) have been detected in nearly all body fluids, the concentration of EGF in tissues is generally low. EGF is a member of a family of growth factors that bind to the same 170 kDa receptor, including TGF-$\alpha$, vaccinia growth factor and amphiregulin17. EGF is initially synthesized as a large (130 kDa) precursor molecule in which the mature, soluble EGF sequence (6 kDa) is located. In vitro, EGF is a mitogen for fibroblasts and endothelial cells and promotes colony formation of epidermal cells in culture. Fibroblast growth factor FGF basic, also called FGF-2 or heparin-binding growth factor 2 (HBGF-2) has been isolated from a variety of sources, including neural tissue and placenta. When isolated from natural sources, FGF basic usually has an apparent molecular mass of about 18 kDa. FGF basic has been shown to stimulate the proliferation of cells of meso-dermal and neuroectodermal origin, including fibroblasts, endothelial cells, astrocytes, oligodendrocytes, neuro-blasts, keratinocytes, bovine lens epithelial cells, osteoblasts, smooth muscle cells, and melanocytes. FGF basic stays membrane-bound as long as there is no signal peptide. FGF-2 has the ability to bind to Heparin. The complex comprising FGF-2 and Heparin does not transmit a biological signal but rather functions by activation of the occupied signaling receptors impacting stability and activity of FGF-2. Platelet-derived growth factor (PDGF) is a glycosylated, disulfide-linked dimer. There are two types of polypeptide, A (16 kDa) and B (14 kDa), with about 50% sequence identity, disulfide linked into three possible dimeric molecules, PDGF-AA, -AB and -BB. The amount of each form depends on the relative expression of the two PDGF polypeptides, which varies by type of cell. The three forms of PDGF have different but overlapping biological activities. There are two structurally related but distinct PDGF receptors, a 170 kDa $\alpha$-receptor and a 190 kDa $\beta$-receptor, each with its own variation in signaling mechanism. Each subunit of PDGF binds one receptor, leading to receptor dimerization. The major source of PDGF in human blood is platelets, where PDGF-AA and -AB are stored and released when platelets are activated. Growth factors such as EGF, FGF-2 and PDGF-BB as used for preparing a membrane according to the invention can be purchased, for example, from R&D Systems.

The polymer membrane which can be used for preparing a coated membrane according to the invention preferably comprises hydrophobic or hydrophilic polymers or both. In one embodiment, the membrane comprises a blend of at least one hydrophilic polymer and at least one hydrophobic polymer. In another embodiment, the membrane comprises hydrophilic copolymers. In yet another embodiment, the membrane comprises hydrophilic copolymers and hydrophobic polymers. In another embodiment, the membrane comprises hydrophilic homopolymers. In a further embodiment, the membrane comprises hydrophilic homopolymers and hydrophobic polymers.

In one embodiment of the invention, the polymer solution used to prepare the membrane comprises hydrophobic and hydrophilic polymers in amounts such that the fraction of hydrophobic polymer in the polymer solution is between 5 and 20 % by weight and the fraction of the hydrophilic polymer is between 2 and 13 % by weight.

In a particular embodiment, the membrane comprises a first hydrophobic polymer component, a second hydrophilic polymer component, and, optionally, a third hydrophobic polymer component.

The expression "cultivation" as used in the context of the present invention comprises such uses as cell attachment or adherence, cell growth and expansion or storage of cells.

**Table I: Abbreviations**

| Abbreviation used | Meaning |
|---|---|
| PES | polyethersulfone |
| FGF | fibroblast growth factor |
| TCPS | tissue culture polystyrene |
| FN | fibronectin |
| EMC | Extracellular Matrix Components |
| Hep (hep) | heparin |
| MSC | Mesenchymal Stem Cells |

The membrane of the present invention can be advantageously used for culturing adherent cells in general. Adherent cells are defined, in the context of the present invention, as cells attaching to a substrate which are to be maintained, expanded, differentiated, stored, etc. The membrane of the invention will be used for culturing, for example, stem cells, including embryonic and adult stem cells, especially mesenchymal stem cells (MSC), fibroblasts, epithelial cells, hepatocytes, endothelial cells, muscle cells, chondrocytes, etc.

In one aspect of the present invention, the modified membranes according to the invention can be advantageously used for the cultivation of renal cells and epithelial cells.

In another aspect of the invention, the membrane is advantageously used for the cultivation (i.e. attachment, growth, expansion and/or storage) of mesenchymal stem cells (MSC). A stem cell as such is a primal undifferentiated cell that has the unique capacity to renew itself and to retain the capability to divide and differentiate into other specialized cell types. Although most cells of the body are committed to conduct a specific function, a stem cell is uncommitted until it receives a signal to develop into a specialized cell. Adult stem cells are distinct from cells isolated from embryos or fetuses and can be found in tissues that have already developed, as in humans after birth. These cells can be isolated from many tissues. However, the most common tissue to obtain adult mesenchymal stem cells is bone marrow, which is located in the centre of large bones. There are different types of stem cells found in the bone marrow, including hematopoietic stem cells and mesenchymal stem cells. It has long been known that hematopoietic stem cells form blood, endothelial stem cells form the vascular system (arteries and veins), and mesenchymal stem cells form bone, cartilage, muscle, fat, and fibroblasts. Stem cells have the ability to act as a repair system for the body, because they can divide and differentiate, replenishing other cells as long as the host organism is alive. Stem cells may contribute to regeneration of damaged livers, kidneys, hearts, lungs and other organs. Stem cells are generally grown in tissue culture dishes or flasks in incubators at body temperature (37°C) under high humidity.

Because of the different characteristics of stem cells, the components of culture media for each type of stem cell may be different for obtaining optimal results. Such media are generally known and have been described in the art.

MSC can be characterized by their shape, i.e. their morphology. MSC show different kinds of morphology depending on the donor, the age of cells (number of doublings), cultivation with different media, incubation with growth factors, and other parameters.

It is an object of the present invention to provide for a membrane which allows the cultivation of cells which have an optimal morphology, i.e. will not deviate from a cell which was not grown under artificial conditions. Generally three different morphologies have been classified in literature from which the spindle shape is regarded as the ideal one as it had been shown to have the highest proliferative potential, whereas the large and flattened shape has the least proliferative potential. Figures 4, 5 and 6 show the morphology of MSC which have been expanded on a standard TCPS flask (Fig. 4), a membrane coated with fibronectin (Fig. 5) and on a membrane which was coated according to the invention with fibronectin, FGF-2 and heparin (Fig. 6). It can be seen from Fig. 6 that the membrane according to the invention allows the growth of the cells in a spindle shaped morphology.

As stated before, it is a goal of the present invention to provide for a membrane for the cultivation of cells which will result in cells which do not differ or differ in as few as possible aspects from cells which were not grown under artificial conditions. Cells can be characterized by surface antigens, so called cluster of differentiation (CD) molecules. CDs are a defined subset of cell surface molecules. They are found on the surfaces of various cells and can be recognized by specific sets of antibodies. These antibodies can be used to identify the cell type, stage of differentiation and activity of a cell. Detection of these surface molecules can be performed with the help of, for example, flow cytometry, immunohistochemistry and western blot. Since no specific marker for MSC is known to date, MSC are phenotypically characterized by a combination of CDs, which MSC are known to express or to not express on the cell surface. Typically, CD31, CD34, and CD45 etc. are not found on MSC, whereas CD29, CD44, CD73, CD90 and many more have been discovered on the surface of MSC. Table II depicts the results for MSC which were harvested from different growth matrices and submitted to flow cytometry. The numbers refer to the portion of cells in % which show the respective clusters or complexes (positive cells). The cells which were harvested from the membrane according to the invention show that the phenotype is typical for MSC (CD45 and HLA-DR below 2%; CD73 and CD90 above 95%. There is no significant difference between the tested matrices.

**Table II**

|  | TCPS flask (% pos. cells) | Membrane coated with FN (% pos. cells) | Membrane coated with FN/Hep/FGF-2 (% pos. cells) |
|---|---|---|---|
| **CD45** | 0.03 | 0.43 | 0.14 |
| **HLA-DR** | 0.13 | 0.55 | 0.47 |
| **CD73** | 99.64 | 97.37 | 99.53 |
| **CD90** | 99.94 | 99.59 | 99.75 |

In a further aspect of the present invention, the performance in culturing cells can be significantly improved by coating, according to the invention, a hollow fiber membrane, and by using said hollow fiber membrane or a bundle thereof in a continuous culture process as an alternative to plate culture techniques. Besides a continuous process, the hollow fiber membrane can of course also be used in a static or semi-continuous process.

The membrane of the invention can be prepared in ways that confer the specific properties to the whole of the membrane, i.e., in case of a hollow fiber membrane for continuous applications, to the outside and/or inside of the hollow fiber membrane.

In a further aspect of the present invention, the membrane also provides a system for cellular co-cultivation of two or more different cell types.

A further aspect of the present invention is that the membrane very well promotes the formation of an optimal cell monolayer in terms of differentiation and integrity. The membrane of the invention provides for the retention of typical cell morphology (Fig. 6), a monolayer is readily formed, and tight junctions can be created. In the context of the present invention, a monolayer refers to a layer of cells in which no cell is growing on top of another, but all are growing side by side and are in many cases touching each other on the same growth surface, even though this is not necessary for all potential applications of the membrane.

The present invention is also directed to a modified membrane as described before and hereafter, wherein the membrane is populated with cells, preferably with adherent cells. The cells preferably form a monolayer. In the context of the present invention, the term "populated" further comprises cells which will grow on top of each other (multiple layers) and monolayers or multiple layers wherein the cells contacting the membrane surface do not all touch each other.

The membrane of the invention can thus be advantageously used, for example,

(a) in tissue culture technology, i.e. for establishing bioartificial implants, such as bioartificial kidneys or livers (see also Atala A, et al. Tissue-engineered autolo-gous bladders for patients needing cystoplasty. Lancet (2006), 367: 1241-6);

(b) for cultivating adherent cells, such as, for example, MSC, smooth muscle cells, skin cells, nerve cells, neuro-glia or endothelial cells in general, or suspension cells, such as hematopoietic stem cells, cord blood cells, neural stem cells, etc. for use in medical therapies via injection of cells, which need to be expanded *in vitro* before being re-implanted into the host;

(c) for expanding and providing cells which serve as producers of bio products such as growth factors, recombinant proteins, cytokines or antibodies, such as monoclonal antibodies;

(d) for preparing cultures of adherent cells, preferably cell monolayer cultures, for studying specific cell types or for studying the influence of any drugs on cells (screening procedures), such as, for example, anti-cancer agents, anti-fungals, antibiotics, anti-virals (including anti-HIV) and anti-parasitic drugs;

(e) or any other application which is based on or requires the culturing expansion or storage of adherent or suspension cells in an *in vitro* system.

The membrane of the invention can have any suitable geometry according to the needs of the intended use, i.e. it can be a flat sheet, a hollow fiber or a bundle of hollow fibers, or can be shaped to form chambers or other geometries desired. The core unit for cell expansion preferably is a hollow fiber-based membrane system allowing sufficient exchange of $O_2$ and $CO_2$, supply of nutrients and removal of waste products (Fig. 7 and 8).

It is one aspect of the present invention to provide a system for the expansion for the culturing of cells comprising a membrane according to the invention. Figure 7 and Figure 8 schematically depict a basic setup for such system for culturing cells. The system comprises a membrane housing, wherein the cells are expanded on a membrane according to the invention, i.e. a bioreactor. Preferably, the membrane is a hollow fiber membrane which is used within a conventional dialyzer housing having the standard outlets and inlets. A bundle of such hollow fiber membranes is introduced into the housing. The system further comprises reservoirs for the supply of fresh culture medium (2, 6), sterile filters (3) through which medium can be added to the system and a pump (4) which transports the medium into the bioreactor. The system may further have specific sites for the sampling of the medium and/or cells which leave the bioreactor (5).

The medium can be present either on the outside or on the inside of the hollow fiber membrane. If the medium is exchanged intra-capillary, the pump (4) will be located in the vicinity of reservoir (6). Fresh medium is added via the reservoir (6). If the medium is exchanged extracapillary the system set-up is as shown in Fig. 7, wherein fresh medium is added via reservoir (2).

The surface of the membrane is designed to enable adhesion and proliferation of cells having the desired properties through the specific surface coating. The advantages of the cultivation of cells inside of hollow fibers is based on the advantageous surface to volume ratio which results in the minimization of medium consumption in the cultivation process, the minimization of space requirements and the minimization of labor as compared to conventional flask or cell stack culture methods. Another advantage of the hollow fiber structure is uniform controlled flow paths.

The membrane of the invention can be used in various kinds of cell expansion or cell culturing devices or systems, such as described, for example, in US 2003/0203478 A1, US 6,150,164 or US 6,942,879.

As mentioned before, a broad variety of membranes can be use for the coating according to the invention.

In a first aspect of the invention, membranes which can be coated according to the invention are prepared from a polymer mixture comprising hydrophobic and hydrophilic polymers in amounts such that the fraction of hydrophobic polymer in the polymer solution used to prepare the membrane is from 5 to 20 % by weight and the fraction of the hydrophilic polymer is from 2 to 13 % by weight. Said at least one hydrophobic polymer is preferably chosen from the group consisting of polyamide (PA), polyaramide (PAA), polyarylethersulfone (PAES), polyethersulfone (PES), polysulfone (PSU), polyarylsulfone (PASU), polycarbonate (PC), polyether, polyurethane (PUR), polyetherimide and copolymers of said polymers, preferably polyethersulfone or a mixture of polyarylethersulfone and polyamide. Said at least one hydrophilic polymer is preferably chosen from the group consisting of polyvinylpyrrolidone (PVP), polyethylene glycol (PEG), polyglycolmonoester, water soluble cellulosic derivates, polysorbate and polyethylene-polypropylene oxide copolymers, preferably polyvinylpyrrolidone.

A membrane which may be preferably used in the context of the present invention comprises, in the polymer solution for preparing the membrane, from 11 to 19 wt.-% of a first polymer selected from the group consisting of polysulfone (PS), polyethersulfone (PES) and polyarylethersulfone (PAES), from 0.5 to 13 wt.-% of a second polymer such as polyvinylpyrrolidone (PVP), from 0 wt.-% to 5 wt.-%, preferably from 0.001 to 5 wt.-% of a polyamide (PA), from 0 to 7 wt.-% of water and, the balance to 100 wt.-%, of a solvent selected from the group consisting of N-methyl-2-pyrrolidone (NMP), which is preferred, N-ethyl-2-pyrrolidone (NEP), N-octyl-2-pyrrolildone (NOP), dimethyl acetamide, dimethyl formamide (DMF), dimethyl sulfoxide (DMSO) and gamma-butyrolactone (GBL).

Preferably, the polyvinylpyrrolidone (PVP) in the polymer solution consists of a blend of at least two homopolymers of polyvinylpyrrolidone wherein one of the homopolymers of polyvinylpyrrolidone (= low molecular weight PVP) has an average relative molecular weight of from about 10,000 g/mol to 100,000 g/mol, preferably about 30,000 g/mol to 70,000 g/mol, and another one of the homopolymers of polyvinylpyrrolidone (= high molecular weight PVP) has an average relative molecular weight of from about 500,000 g/mol to 2,000,000 g/mol, preferably about 800,000 g/mol to 2,000,000 g/mol. Examples of such PVP homopolymers are PVP K85, a high molecular weight PVP having a molecular weight of about 825,000 Da, and PVP K30, a low molecular weight PVP having a molecular weight of about 66,800 Da. In a preferred embodiment of the present invention, the polymer solution for preparing the membrane comprises from 0.5 to 5 wt.-percent of a high molecular weight PVP and from 1 to 8 wt.-% of a low molecular weight PVP.

Methods for preparing such membranes are described in detail, for example, in US-A 4,935,141, US-A 5,891,338 and EP 1 578 521 A1, all of which are incorporated herein by reference. Examples for this type of membrane, which can be effectively treated according to the present invention, are Gambro Polyflux™ membranes (polyarylethersulfone/PVP/polyamide), which are currently used in commercial products, such as, for example, Polyflux™ L and H series; Gambro Arylane™ membranes (poly(aryl)ethersulfone/PVP); DIAPES™ or PUREMA™ membranes (poly (aryl) ethersulfone/PVP) or any other commercial dialysis membranes based on blends of hydrophilic and hydrophobic polymers, e.g. blends comprising PVP and PES or polysulfone.

In a second aspect of the present invention, the polymer solution used to prepare the membrane which can be coated according to the invention comprises from 12 to 15 wt.-% of polyethersulfone or polysulfone as hydrophobic polymer and from 5 to 10 wt.-% of PVP, wherein said PVP consists of a low and a high molecular PVP component. The total PVP contained in the spinning solution consists of from 22 to 34 wt.-%, preferably of from 25 to 30 wt.-%, of a high molecular weight (> 100 kDa) component and from 66 to 78 wt.-%, preferably from 70 to 75 wt.-% of a low molecular weight (<= 100 kDa) component. Examples for high and low molecular weight PVP are, for example, PVP K85/K90 and PVP K30, respectively. The polymer solution used in the process of the present invention preferably further comprises from 66 to 86 wt.-% of solvent and from 1 to 5 wt.-% suitable additives. Suitable additives are, for example, water, glycerol and/or other alcohols. Water is especially preferred and, when used, is present in the spinning solution in an amount of from 1 to 8 wt.-%, preferably from 2 to 5 wt.-%. The solvent used in the process of the present invention preferably is chosen from N-methylpyrrolidone (NMP), dimethyl acetamide (DMAC), dimethyl sulfoxide (DMSO), dimethyl formamide (DMF), butyrolactone and mixtures of said solvents. NMP is especially preferred. The center fluid or bore liquid which is used for preparing the membrane comprises at least one of the above-mentioned solvents and a precipitation medium chosen from water, glycerol and other alcohols. Most preferably, the center fluid consists of 45 to 70 wt.-% precipitation medium and 30 to 55 wt.-% of solvent. Preferably, the center fluid consists of 51 to 57 wt.-% of water and 43 to 49 wt.-% of NMP.

Methods for preparing such membranes are disclosed in detail in EP 2 113 298 (A1), expressly incorporated herein by reference. Examples for this type of membrane, which can be treated effectively according to the present invention, are, for example, the Gambro U9000® and Revaclear™ membrane and derivatives thereof. It is also possible to use, in the context of the present invention, membranes which are currently used in commercial products, such as, for example, the Fresenius FX™-class membranes (Helixone™ membranes) or Optiflux™ type membranes) or other commercial dialysis membranes based on blends of hydrophilic and hydrophobic polymers, e.g. blends comprising PVP and PES or polysulfone.

In a third aspect of the present invention, the polymer solution used to prepare the membrane which can be coated according to the invention comprises from 11 to 19 wt.-% of a first polymer selected from the group consisting of polysulfone (PS), polyethersulfone (PES) and polyarylethersulfone (PAES), from 0.5 to 13 wt.-% of a second polymer such as polyvinylpyrrolidone (PVP), from 0.001 to 20 wt.-% of a polyurethane (PU), from 0 to 7 wt.-% water and a solvent selected from the group consisting of N-methyl-2-pyrrolidone (NMP), N-ethyl-2-pyrrolidone (NEP), N-octyl-2-pyrrolildone (NOP), dimethyl acetamide, dimethyl formamide (DMF), dimethyl sulfoxide (DMSO) and gamma-butyrolactone (GBL), adding up to 100 wt.-%. Said first polymer is preferably present in the polymer solution in an amount of from 13 to 14 wt.-%, especially preferably in an amount of from 13.6 to 14 wt.-%. Polyethersulfone (PES) and polyarylethersulfone (PAES) are preferably used for preparing a membrane of the invention. Preferably, the polyvinylpyrrolidone (PVP) in the polymer solution consists of a blend of at least two homopolymers of polyvinylpyrrolidone wherein one of the homopolymers of polyvinylpyrrolidone (= low molecular weight PVP) has an average relative molecular weight of about 10,000 g/mol to 100,000 g/mol, preferably about 30,000 g/mol to 70,000 g/mol, and another one of the homopolymers of polyvinylpyrrolidone (= high molecular weight PVP) has an average relative molecular weight of about 500,000 g/mol to 2,000,000 g/mol, preferably about 800,000 g/mol to 2,000,000 g/mol. Examples for such PVP homopolymers are PVP K85, a high molecular weight PVP having a molecular weight of about 825,000 Da, and PVP K30, a low molecular weight PVP having a molecular weight of about 66,800 Da. In a preferred embodiment of the present invention, the polymer solution for preparing the membrane comprises from 0.5 to 5 wt.-% of a high molecular weight PVP and from 1 to 8 wt.-% of a low molecular weight PVP. The water content of the spinning solution preferably is from 1 to 5 wt.-%, more preferably about 3 wt.-%. Various solvents can be used for preparing a membrane of the invention, such a s N-methyl-2-pyrrolidone (NMP), N-ethyl-2-pyrrolidone (NEP), N-octyl-2-pyrrolidone (NOP), dimethyl acetamide, dimethyl formamide (DMF), dimethyl sulfoxide (DMSO) or gamma-butyrolactone (GBL) and mixtures thereof. The solvent will be present in an amount to add up to 100 wt.-% of the polymer solution. The content of the solvent in the polymer solution preferably is from 60 to 80 wt.-%, more preferably from 67 to 76.4 wt.-%.

The membranes which can be coated according to the invention can be prepared, for example, in flat sheet or hollow fiber geometry.

In one embodiment, the membranes which can be coated according to the invention have an asymmetric structure. In the case of hollow fibers, there is a thin separation layer on the inner side of the fibers. The structure or morphology of the membrane of the invention may otherwise vary without significant impact on its performance regarding cell adhesion and proliferation. The membranes may have, for example, a 3-layer structure or a sponge-like structure or a foam-like structure. In one embodiment, the membrane of the invention is further characterized by the smoothness or low roughness of the cell adhesion side.

In one embodiment, the hydraulic permeability of a membrane which can be coated according to the invention may vary from about $0.1 \cdot 10^{-4}$ cm$^3$ to $200 \cdot 10^{-4}$ cm$^3$/(cm$^2$ bar sec), e.g. $0.1 \cdot 10^{-4}$ cm$^3$ to $10 \cdot 10^{-4}$ cm$^3$/(cm$^2$ bar sec), or even $0.1 \cdot 10^{-4}$ cm$^3$ to $5 \cdot 10^{-4}$ cm$^3$/(cm$^2$ bar sec). In order to achieve such hydraulic permeability without getting defects in the membrane structure, the viscosity of the polymer solution usually will be in the range of from 2,500 centipoise (cP) to 200,000 cP, or even from 10,900 cP to 25,600 cP for hollow fiber production. For flat sheet membrane production the viscosity generally will be in the range of from 2,500 cP to 500,000 cP, or even from 4,500 cP to 415,000 cP.

For preparing the membranes which can be coated according to the invention, the polymers are dissolved in the solvent at constant temperature and pressure. Degassing of the polymer solution is performed in a drying oven creating a vacuum (approximately 100 mbar). The temperature of the polymer solution may vary over a relatively broad range. It is advantageous to choose a temperature in the range of from ambient temperature to 60°C.

For preparing a flat sheet membrane which can be coated according to the invention, the final polymer solution is cast as a uniform film onto a smooth surface such as a glass slide which acts as a supporting area, by utilizing a special coating knife. The velocity of casting the polymer film can vary over a relatively broad range. A velocity between 10 and 20 mm/s may be appropriate. In an exemplary lab-scale process, the polymer solution first is applied steady-going onto the glass slide using a syringe. It is important to work bubble free. The coating knife with a defined gap height is driven with constant velocity, creating a uniform polymer film. For a good thickness distribution, a coating knife having a uniform gap is advisable.

A hollow fiber membrane used in the invention is further characterized by having an inner diameter of from 50 to 2,000 μm, preferably of from 50 to 1,000 μm, and more preferably from 100 to 500 μm. The hollow fiber membrane has a wall thickness of from 10 to 200 μm, preferably of from 20 to 100 μm, and more preferably from 25 to 55 μm.

The thickness of a flat sheet membrane which are used according to the invention may vary between 20 μm and 200 μm. A thickness of 35 μm to 50 μm may be especially advantageous for most applications.

In one embodiment of the invention, the precipitation bath comprises H$_2$O in an amount of from 30 to 100 wt.%, preferably in an amount of from 56 to 66 wt.-%, and a solvent, such as NMP, in an amount of from 0 to 70 wt.-%, preferably from 34 to 44 wt.-%. The temperature of the precipitation bath can be varied over a relatively broad range. It may be advantageous to apply a temperature between 0°C and 80°C, or between 30°C and 50°C. The precipitation time can also be varied. As an example, the precipitation time may be about five minutes. The precipitation bath preferably consists of $H_2O$ and a solvent. The bath preferably comprises $H_2O$ in amount of from 30 wt.-% to 100 wt.-%, and a solvent selected from N-methyl-2-pyrrolidone (NMP), N-ethyl-2-pyrrolidone (NEP), N-octyl-2-pyrrolildone (NOP), dimethyl acetamide, dimethyl formamide (DMF), dimethyl sulfoxide (DMSO) or gamma-butyrolactone (GBL) and mixtures thereof in an amount of from 70 wt.-% to 0 wt.-%. In one embodiment of the invention, the precipitation bath comprises $H_2O$ in an amount of from 56 to 66 wt.-%, and a solvent in an amount of from 34 to 44 wt.-%. NMP is an especially suitable solvent in the context of the present invention.

The precipitated membrane is then stored in a non-solvent until the membrane is cut. After cutting, the membrane is washed, dried and sterilized.

The thickness of a flat sheet membrane which can be coated according to the invention may vary between 15 $\mu$m and 200 $\mu$m. A thickness of 35 $\mu$m to 50 $\mu$m may be especially advantageous for most applications.

The membranes which can be coated according to the invention can also be prepared in hollow fiber geometry. For preparing such hollow fiber membranes, the solution is pumped through a spinning die and the liquid hollow fiber is formed. The solvent concentration in the center results in an open structure at the inner side of the membrane. The smallest pores are directly at the inner side of the membrane. When in use, the selective layer at the inside is in direct contact with cell medium.

In one embodiment, the precipitation bath consists of water. The temperature of the precipitation bath may be varied over a broad range, but ambient temperature up to about 40°C is advantageously used in the process. The distance between the die and the precipitation bath is in the range of from 0 to 100 cm, e.g. from 50 to 100 cm. The die (spinneret) temperature can also be varied. Temperatures between 20 and 80°C can be used. It may be advantageous to apply temperatures between 40 and 55°C. The spinning speed may be chosen to be in the range of from 5 to 80 m/min, e.g. from 11 to 40 m/min.

The dimensions of a hollow fiber membrane which can be coated according to the invention may be varied depending on the intended use of the membrane. The inner diameter generally is in the range of from 50 to 2,000 $\mu$m. For many applications, an inner diameter of from 100 to 950 $\mu$m may be advantageous. The wall thickness generally is in the range of from 25 to 55 $\mu$m.

The resulting Lp for a membrane which can be coated according to the invention is in the range of from $0.1 \cdot 10^{-4}$ to $200 \cdot 10^{-4}$ cm$^3$/(cm$^2 \cdot$bar$\cdot$s), e.g. from $0.1 \cdot 10^{-4}$ to $10 \cdot 10^{-4}$ cm$^3$/(cm$^2 \cdot$bar$\cdot$s), or even from $0.1 \cdot 10^{-4}$ to $5 \cdot 10^{-4}$ cm$^3$/(cm$^2 \cdot$bar$\cdot$s). In another embodiment of the invention, the Lp of the membranes is in the range of from $2 \cdot 10^{-4}$ to $18 \cdot 10^{-4}$ cm$^3$/(cm$^2 \cdot$bar$\cdot$s). In still another embodiment of the invention, the Lp of the membranes in the range of from $5 \cdot 10^{-4}$ to $15 \cdot 10^{-4}$ cm$^3$/(cm$^2 \cdot$bar$\cdot$s). That is, membranes which are to be used for cell culture can be so-called low flux membranes.

There are two ways for producing membranes which can be coated according to the invention which may be referred to as "wet" and "dry". In case "wet" membranes are prepared, the membranes have to be dried separately in a tube or oven after they have been prepared. To this end, bundles of fibers (for example, from 30 to 15,000 fibers) are placed in a plastic or metal container. Hot air is passed through this container to dry the membranes. The second way is the so-called "online drying" which is an efficient way to directly prepare dry hollow fibers on a spinning machine. Both procedures are applicable to arrive at membranes which may be treated and used according to the invention.

The present invention is also directed to providing a process for preparing a modified or coated membrane according to the invention.

In one aspect of the present invention, the process comprises the steps of

(a) incubating a mixture of a growth factor, a glycoprotein and a glycosaminoglycan;
(b) coating the surface of a membrane with the mixture by contacting the mixture and the membrane;
(c) removing the supernatant and optionally rinsing the membrane.

In another aspect of the invention, the process comprises the steps of

(a) incubating a membrane with a mixture of a growth factor, a glycoprotein and a glycosaminoglycan;
(b) removing the supernatant and optionally rinsing the membrane.

In one aspect of the present invention, the membranes are washed or rinsed prior to the coating process. This may be done, for example, with a NaCl solution at room temperature.

The coating solution comprises a growth factor (or a mixture of variants thereof or various growth factors), a glycosaminoglycan and a glycoprotein. In a preferred embodiment, the coating solution comprises a mixture of FGF-2, heparin and fibronectin.

The concentration of the growth factor, the glycosaminoglycan and the glycoprotein may vary over a certain range and depending on which specific compound is being used.

In one aspect of the invention, the concentration of FGF-2 should lie in the range of from 50 ng/ml to 500 ng/ml, preferably in the range of from 100 ng/ml to 300 ng/ml. It is especially advantageous to use a FGF-2 concentration of from 150 ng/ml to 250 ng/ml.

In a further aspect of the present invention, the concentration of fibronectin should be in the range of from 1 μg/ml to 200 μg/ml, preferably in the range of from 5 μg/ml to 100 μg/ml. It is especially advantageous to use a fibronectin concentration of from 10 μg/ml to 50 μg/ml.

The concentration of heparin should be in the range of from 1 μg/ml to 200 μg/ml, preferably in the range of from 5 μg to 100 μg. It is especially advantageous to use a heparin concentration of from 10 μg/ml to 50 μg/ml.

The coating solution is generally prepared by incubating the growth factor, the fibronectin and the heparin at a temperature of between 0°C and 24°C for a time of between 2 and 24 hours, preferably at a temperature of between 2°C and 12°C for 6 to 18 hours.

In another aspect of the invention, the coating solution is incubated according to step (a) for a time of between 2 and 24 hours at a temperature of between 0°C and 24°C. In a preferred aspect of the invention, the incubation will be done at a temperature of between 2°C to 12°C. In another preferred aspect of the invention the incubation will be done for a time of between 3 hours and 24 hours, preferably between 8 hours and 15 hours.

In yet another aspect of the invention, the coating solution is removed after incubation, preferably by aspiration, and the coated membrane is dried before cells are added to the coated membrane.

The coated membranes may then be used directly for culturing cells of different types, preferably adherent cells. The membranes of the invention exhibit growth characteristics superior to tissue culture polystyrene (TCPS) plates which represent today's gold standard for cell expansion using culture flasks or cell stacks.

For the cultivation of cells, the process further comprises

(d) incubating the membrane of step (c)or (b) with the cells at an appropriate temperature for attaching the cells to the membrane surface;
(e) optionally exchanging the medium during the proliferation of the cells at an appropriate interval;
(f) detaching the cells from the membrane, preferably with detachment enzymes;
(g) optionally re-seeding the cells on a new membrane and repeating steps (d) to (f)or (d) to (g);
(h) harvesting the cells;
(i) optionally determining the cell count of the detached cells and/or characterizing the detached cells.

The cells are generally seeded in a medium which is appropriate for the given cell type. For example, MSC were cultured in an alpha-MEM cell culture medium with addition of fetal calf serum (FCS) and penicillin-streptomycin according to methods known in the art.

The temperature used for the cultivation can also be adapted to the specific cell type. In general, the temperature will be in the range of between 30°C to 38°C, preferably between 35°C and 37°C. It may prove advantageous to cultivate the cells in a humidified atmosphere with about 5% $CO_2$.

Re-seeding the cells onto another membrane can be referred to as passage or sub-cultivation of the cells. This step comprises detaching the cells from the surface by using detachment enzymes and transferring the cells which are now in suspension to another cell culture container where they can re-attach to the surface of the coated membrane and proliferate.

For detachment of the cells, the cell culture media is removed, cells are rinsed with an appropriate buffer, such as, for example, PBS (phosphate-buffered saline). Afterwards, wash solution is removed and detachment enzyme is added. Cells are then incubated with the detachment enzyme (incubation time respective to enzyme used). The detached cells can then be counted, for example by using a CASY counter, centrifuged and aliquots can be transferred into a new medium.

In order to maximize the cells' proliferation surface, the cells should be re-distributed (re-seeded) when they reach a certain density, at least when they reach confluency. The appropriate time for re-seeding can be determined by monitoring cell growth regularly, for example via microscope.

The expression "confluency" is commonly used as a measure of the number of the cells in a cell culture dish or a flask and refers to the coverage of the dish or the flask by the cells. For example, 100 per cent confluency means the dish is completely covered by the cells and there is no more room left for the cells to grow where as 50 per cent confluency means roughly half of the dish is covered and there is room for cells to grow.

In one aspect of the invention, the cells are cultivated in a bioreactor system (Fig. 7 and 8 and below). In this case, the cells (or detached cells ready for re-seeding) may be loaded into a hollow fiber bioreactor. To this end, the cell suspension may be loaded into a syringe and flushed into the bioreactor. The medium should be exchanged after the cells are attached to the membrane, for example after 12 to 24 hours.

The doubling time (dt) is a measure of growth behavior of cells and is defined as follows:

$$dt = \frac{\ln 2}{\ln FE} \times t(hours) \, ,$$

wherein FE represents "Fold Extension", which is defined as the ratio of the number of cells after expansion and the number of cells before expansion.

The membranes of the invention show cell expansion rates, re-attachment efficiency of cells onto membranes, and characteristics of the cells' post expansion including morphology control at least similar and often superior to tissue culture polystyrene (TCPS), as exemplarily shown in tests performed with mesenchymal stem cells (Examples 3 and 4, Figures 2 and 3).

A further aspect of the invention is a cell culturing device comprising a membrane which is coated according to the invention. Examples of cell expansion or cell culturing devices or systems which can be modified to comprise the membrane of the invention are disclosed in US 2003/0203478 A1, US-A 6,150,164, or US-A 6,942,879, all incorporated herein by reference. The device can comprise a stack of flat sheet membranes of the invention or a bundle of hollow fiber membranes of the invention.

In one embodiment of the device, the membrane forms an interface between two fluid compartments of the device. The device can be similar in construction to commercially available filtration devices used, for example, in hemodialysis or haemofiltration.

An exemplary device comprises two compartments separated by a semipermeable membrane mounted in a casing, a first internal compartment fitted with two accesses and a second external compartment comprising one or two accesses, both compartments being also separated by a potting compound, based on an appropriate adhesive compound, intended for forming, as applicable, (i) a cylindrical partition separating both compartments of said device containing a semi-permeable membrane of the hollow fiber bundle type as defined above or (ii) a tight seal in said device including a semipermeable membrane of the sheet membrane type as defined above.

Another exemplary device comprises a plurality of hollow fiber membranes, contained within an outer shell, and configured so that fluid within a space external to the hollow fibers (i.e., an extracapillary compartment) is segregated from fluid passing through the hollow fibers and their corresponding orifices. Additionally, the device includes two manifold end chambers within the outer shell on opposite ends of the device. Each of the two mouths of a hollow fiber connects to a different end chamber. The end chambers and the extracapillary compartment are separated by the semipermeable membranes of the hollow fibers. The composition within the extracapillary compartment can be controlled, to a certain extent, by the molecular weight cut-off, or pore size, of the membranes of the hollow fibers.

In one mode of operating the device, cells are grown in the extracapillary compartment while a nutrient medium is passed through the hollow fibers. Medium may be passed through the extracapillary or intracapillary compartment. In another mode of operating the device, cells are grown in the intracapillary space (i.e. lumen) of the hollow fibers while a nutrient medium is passed through the extracapillary and/or intracapillary compartment. The semipermeable nature of the hollow fibers allows nutrients, gas and cell waste products to pass through the walls of the hollow fibers while blocking cells from doing the same. Examples of such a device have been described above and in Figures 7 and 8.

Shell-and-tube type bioreactors provide several advantages. For adherent cells, the use of several hollow fibers provides, within a relatively small volume, a large amount of surface area upon which the cells can grow. This large amount of surface area also facilitates localized distribution of nutrient media to the growing cells and ready collection of cell waste products. Shell-and-tube type bioreactors enable the growth of cells at much higher density rates than is possible with other cell culture devices. They can support cell densities greater than $10^8$ cells per milliliter, whereas other cell culture devices are typically limited to densities around $10^6$ cells per milliliter.

A further aspect of the invention provides a device for the extracorporeal treatment of body fluids, comprising cells and a membrane of the invention. In one embodiment, the cells are adherent cells which form a confluent layer on a surface of the membrane, for instance the surface of the lumen of a hollow fiber membrane of the invention, or the outer surface of a hollow fiber membrane of the invention. For the rest, the design of the device can be similar to the design described above for the cell culturing device. The body fluid to be treated is conducted through a fluid space of the device where it passes over the cell layer, allowing the cells to extract components from the body fluid, to metabolize components of the body fluid, or to segregate components into the body fluid.

**Examples**

The assessment of the suitability and efficiency of the membranes of the invention was based, in general, on the following principal characteristics: cell expansion rate, re-attachment efficiency of cells onto membranes, and characteristics of the cells' post expansion including morphology control. MSC were chosen as an example for the in depth analysis of the performance of membranes of the invention for cell culture, even though other cell types, such as renal cell or epithelial cells, can equally well be cultured in the membranes according to the invention. Therefore, the Examples are not limiting with regard to the cell types which can be cultivated on the membranes according to the invention.

**1. Methods**

1.1 Preparation of hand bundles, mini-modules, filters and

flat sheet inserts

1.1.1 Hand bundles

The preparation of the membrane bundle after the spinning process is necessary to prepare the fiber bundle in an adequate way for succeeding performance tests. The first process step is to cut the fiber bundles to a defined length of 23 cm. The next process step consists of melting the ends of the fibers. An optical control ensures that all fibers are well melted. Then, the ends of the fiber bundle are transferred into a potting cap. The potting cap is fixed mechanically and a potting tube is put over the potting caps. Afterwards, the potting is done with polyurethane. After the potting is has to be ensured that the polyurethane can harden for at least one day. In the next process step, the potted membrane bundle is cut to a defined length and to open the ends of the fibers. The last process step consists of an optic control of the fiber bundle. During this process step, the following points are controlled: (i) quality of the cut (is the cut smooth or are there any damages of the knife), (ii) quality of the potting (is the number of open fibers of the spinning process reduced by fibers that are potted or are there any visible voids where the there is no polyurethane). After the optical control, the membrane bundles are stored dry before they are used for the different performance tests.

1.1.2 Preparation of mini-modules

Mini-modules [= fiber bundles in a housing] are prepared with related process steps. The mini-modules are needed to ensure a protection of the fibers and a very clean manufacturing. The manufacturing of the mini-modules differs in the following points: (i) the fiber bundle is cut to a defined length of 20 cm; (ii) the fiber bundle is transferred into the housing before the melting process; (iii) the mini-module is put into a vacuum drying oven over night before the potting process.

1.1.3 Preparation of filters

The filter comprises about 8.000 to 15.000 fibers with an effective surface area of 0.9 to 1.7 $m^2$. A filter is characterized by a cylindrical housing with two connectors for the supplying culture medium fluid and applied caps on both sides, each with one centered connector. The manufacturing process (after winding) can be split up into the following main steps: (i) the cut (length of approx. 30 cm) bundles are transferred into the housing with a special bundle claw; (ii) both ends of the bundles are closed by a closing process; (iii) the fibers are potted into the housing with Polyurethane (PUR); (iv) the ends are cut to open the fibers, wherein a smooth surface is required; (v) the ends are inspected visually for closed fibers or imperfections in the PUR block; (vi) the caps are glued to the connectors; (vii) final treatment: rinsing, integrity testing, final drying; (viii) packaging in special bags for further steps (e.g. irradiation)

1.1.4 Preparation of flat sheet inserts

Flat membranes are immobilized on glass plates. Polyurethane functioning as glue for inserts is evenly distributed on a plate. The inserts are gently immersed in polyurethane and immediately glued onto the respective membrane. Inserts are weighed down with a glass and iron plate and dried for 16 to 18 hours. Flat membrane inserts are cut out and welded into sterilization bags. Finally, inserts may be sterilized in an autoclave at 121°C.

1.1.5 Hydraulic Permeability (Lp) of hand bundles and mini-modules

The hydraulic permeability of a membrane bundle is determined by pressing an exact defined volume of water under pressure through the membrane bundle, which has been sealed on one side, and measuring the required time. The hydraulic permeability can be calculated from the determined time, the effective membrane surface area, the applied pressure and the volume of water pressed through the membrane. From the number of fibers, the fiber length as well as the inner diameter of the fiber, the effective membrane surface area is calculated. The membrane bundle has to be wetted thirty minutes before the Lp-test is performed. For this purpose, the membrane bundle is put in a box containing 500 ml of ultrapure water. After 30 minutes, the membrane bundle is transferred into the testing system. The testing system consists of a water bath that is tempered at 37 °C and a device where the membrane bundle can be implemented mechanically. The filling height of the water bath has to ensure that the membrane bundle is located underneath the water surface in the designated device. To avoid that a leakage of the membrane leads to a wrong test result, an integrity test of the membrane bundle and the test system has to be carried out in advance. The integrity test is performed by pressing air through the membrane bundle that is closed on one side of the bundle. Air bubbles indicate a leakage of the membrane bundle or the test device. It has to be checked if the leakage can be associated with the wrong implementation of the membrane bundle in the test device or if a real membrane leakage is present. The membrane bundle has to be discarded if a leakage of the membrane is detected. The applied pressure of the integrity test has to be at least the same value as the applied pressure during the determination of the hydraulic permeability in order to ensure, that no leakage can occur during the measurement of the hydraulic permeability because of a too high-applied pressure.

### 1.1.6 Diffusive Permeability of hand bundles

Diffusion experiments with isotonic chloride solution as well as phosphate diluted in dialysis fluid (100 mg/l) are carried out to determine the diffusion properties of a membrane. A hand bundle is put in a measuring cell. The measuring cell allows passing the particular solution at the inside of the hollow fiber. Additionally, the measuring cell is filled completely with water and a high cross flow of distilled water is set to carry away the particular ions that pass the membrane cross section from the inside of the hollow fiber to the outside. By adjusting the pressure ratios correctly, a zero filtration is aimed for, so that only the diffusion properties of the membrane are determined (by achieving the maximum concentration gradient of the particular ion between the inside of the hollow fiber and the surrounding of the hollow fiber) and not a combination of diffusive and convective properties. A sample from the pool is taken at the beginning and a sample of the retentate is taken after 10 and 20 minutes. The chloride samples are then titrated with silver nitrate solution to determine the chloride concentration. The phosphate samples are analyzed photometrically. From the concentrations determined, the effective membrane surface area A and the flow conditions, the permeability P, of chloride or phosphate, respectively, can be calculated according to the following equation (2):

$$P_x\ [10^{-4}cm/s]\ =\ [Q_B/60/A]*ln[(c_A-c_D)/c_R]*10^4 \qquad\qquad (2)$$

with

P = diffusive permeability [cm/s]
c = concentration [mmol]
A = effective membrane surface [cm$^2$]

indices:

x = substance (here: chloride or phosphate, respectively)
A = starting concentration (feed)
D = dialysate
R = retentate

$Q_B$ = blood flow [ml/min]

### 1.1.7 Sieving coefficient for myoglobin in aqueous solution (hand bundle)

The Sieving Coefficient experiments in aqueous solution of myoglobin and albumin are performed using two different experimental set-ups with separate solutions. As a first test the sieving coefficient of myoglobin is determined.
The concentration of myoglobin dissolved in PBS buffer is 100 mg/L. The expiry date of the aqueous solution is between 4 and 8 weeks. The solution has to be stored in the refrigerator. Prior to the Sieving Coefficient experiment, Lp-test is done using the method described earlier. The myoglobin sieving coefficient experiment is run in single pass whereas testing conditions are defined as follows:

The intrinsic flow rate ($J_v$ in cm/s) and wall shear rate ($\gamma$. in $s^{-1}$) are fix whereas the blood flow ($Q_B$) and filtration rate (UF) is calculated (see equation (4) + (5)):

$$Q_B \ [\text{ml/min}] = \gamma * n * \pi * di^3 * 60/32 \qquad (4)$$

$$UF \ [\text{ml/min}] = J_V * A * 60 \qquad (5)$$

with

n = amount of fibers
$d_i$ = inner diameter of fiber [cm]
$\gamma$ = shear rate [$s^{-1}$]
A = effective membrane surface [$cm^2$]

whereas A is calculated according to equation (1):
Testing a hand bundle or a mini-module the shear rate is set to 500 $s^{-1}$ and the intrinsic flow rate is defined to be $0.38 \cdot 10^{-04}$ cm/s.

The first samples are taken after 15 minutes (pool, retentate, and filtrate) and a second time after 60 min. At the end, the test-bundle is rinsed for some minutes with PBS-buffer then the test is stopped.

### 1.2 Cell Culture

If not stated otherwise, MSCs were cultured in $\alpha$-MEM cell culture medium (GIBCO Invitrogen, USA) with addition of 10% foetal calf serum (FCS) and 1% Penicillin-Streptomycin (PS) (10000 units/ml penicillin G sodium and 10000 $\mu$g/ml streptomycin sulfate in 0.85% saline; GIBCO Invitrogen, USA) at 37°C in an humidified atmosphere in an incubator (5% $CO_2$).
This media composition is referred to as standard medium (SM).

### 1.2.1 Detachment of cells

### 1.2.1.1 Trypsin

Typsin is the enzyme most often used to detach cells from surfaces or from their extracellular matrix. Trypsin activ-tiy has to be stopped using serum-containing media. Trypsin-EDTA (0.25% Trypsin with 1 mM EDTA) was purchased from GIBCO Invitrogen, USA.

### 1.2.1.2 Accutase

Accutase is a cell detachment solution of proteolytic and collagenolytic enzymes and is a smooth and gentle but effective alternative to porcine Trypsin for detaching adhering cells. An inactivation of Accutase is not necessary due to its self-digesting activity and the re-attachment of cells is increased following treatment. Accutase in PBS with 0.5mM EDTA was supplied by PAA Laboratories, Austria.

1.3 Substrates (matrices)

1.3.1 Cell culture plastic

T75 flasks, 6-well and 24-well plates cell culture plastic were obtained from BD Falcon, USA. Flasks and plates are made of vacuum gas-plasma modified polystyrene plastic. Inserts for the plates were prepared from the membranes prepared.

1.3.2 Membranes

Substrates used for the cell expansion were based on membranes which are available from Gambro Lundia AB, such as, for example, Polyflux® L or Polyflux® H membranes based on a mixture of polyethersulfone, polyvinylpyrrolidone and polyamide. The Polyflux membrane is prepared from a polymer solution consisting of (in wt-%) NMP (76.4%), polyethersulfone (13.55%), polyamide (0.05%), polyvinylpyrrolidone K85 (2%), polyvinylpyrrolidone K30 (5%) and water (3%). The preparation of the membrane is according to the state of the art or as described, for example, in EP 0 305 787 A1. Another type which was used for the experiments performed is the U9000® membrane (Gambro Lundia AB). The polymer solution from which the membrane is prepared consists of (in wt-%) NMP (76.4%), polyethersulfone (13.6%), polyvinylpyrrolidone K85 (2%), polyvinylpyrrolidone K30 (5%) and water (3%). The preparation of a membrane from said polymer solution is well known in the art and is otherwise described, for example, in EP 1 578 521 A1. Geometries used were, on the one hand, flat sheet membranes and, on the other hand, hollow fiber bioreactors with a surface area of 0.2 $m^2$. For cell adhesion the membrane surface had to be in situ modified as otherwise no cell adhesion would occur.

1.4 Cell counter

Cell numbers were measured by using a modified coulter cell counter system model (CASY TTC) produced by Schaerfe Systems, Germany. For cell measurements, the respective volume of cell suspension was added to 10 ml CASYton and then measured on cell numbers by CASY counter.

1.5 Fluorescence-activated cell-sorting (FACS)

FACS analyses were carried out with a FACS Calibur flow cytometer from Becton and Dickinson. For data analysis, CellQuest software was used. Antibodies were purchased from BD Pharmingen. Flow cytometry is defined as the measurement of the cellular and fluorescent properties of particles in suspension as they pass by a laser or other light source. Labeling of cells in flow cytometry is done using antibodies specific to particular subclasses of cells. Most important cell-type specific antibodies have been given a cluster differentiation (CD) number by international convention. Cells were harvested and the cell suspension was collected for centrifugation for 5 min at 300 g at RT. The supernatant was aspirated prior to re-suspension of the cell pellet in 0.5 ml blocking buffer (10% human serum in BD CellWash) for a 75 $cm^3$ flask. Cell numbers were determined by CASY counter and adjusted to a concentration of about $10^6$ cells/ml. Cells were then incubated for 30-45 min at 4°C in a fridge, on ice and in the dark. 2 µl antibodies were added to 50 µl cell suspension for CD45, CD73, CD 90 and HLA-DR. Thereafter, the suspension was carefully mixed and cells were incubated for 20 minutes at RT in darkness. After that, the samples were washed with 1 ml FACS buffer (2% heat-inactivated FCS in BD CellWash). Following centrifugation for 5 min at RT and 300 g, supernatant was removed and the cell pellet was resuspended in 300 µl staining buffer (BD Cell fix (10x); to be diluted with sterile water 1:10) and stored at 4°C in the dark until analysis.

1.6 R&D Quantikine® ELISA kits

For analysis of growth factor concentrations in collected cell culture supernatants, ELISA Quantikine® Human Immunoassay Kits for EGF, FGF-2, IGF-1 and PDGF-BB supplied by R&D systems were used. The Quantikine® Human Immunoassay Kits comprise an ELISA plate which is already pre-coated with antibodies and all reagents necessary for the assay. Standards, blanks and controls were assayed according to the specified protocol described in the respective ELISA manual supplied by R&D systems.

1.7 Scanning electron microscopy (SEM)

A scanning electron microscope (SEM) is a type of electron microscope capable of producing high-resolution images of a sample surface and so it's possible to investigate surface morphology. SEM creates magnified images (resolution according to device and probe from 20 to 50 nm) by using electrons instead of light waves. For SEM analysis a special sample preparation is necessary (all volumes are given for cell culture inserts): At first, samples were rinsed 3 times for 5 min with 2 ml 0.9% NaCl before fixing the samples with a 2% glutardialdehyde solution for at least 24 hrs at 4°C. Samples were rinsed three times with 2 ml distilled water. Specimen were lyophilized or air-dried.

**Example 1**

In situ modification with fibronectin (FN), heparin (Hep) and FGF-2

The substrate (U9000® from Gambro) was prepared according to the above described method (1.1.4) as a flat sheet membrane. The membrane was modified with a complex comprising 67.5 μg/insert FN, 20 μg/ml Hep and 200 ng/ml FGF-2. Heparin was supplied by ratiopharm in stock solution of 25,000 I.E. in 5 ml corresponding to 33.3 μg/μl. Formation of a complex of heparin, FGF-2 and FN was obtained by incubation over night at 4°C. Prior to coating, flat sheet membrane substrates were washed 3 times with 0.9% NaCl for 10 min at room temperature (2 ml/insert and 4 ml/well) and 2 ml of coating solution was then added for incubation over night at 4°C. The next day, the solution was aspirated and membranes were allowed to dry for about 2 hours before cells were added.

**Example 2**

In situ modification with fibronectin (FN)

Prior to coating, flat sheet membrane substrates were washed 3 times with 0.9% NaCl for 10 min at room temperature (2 ml/membrane insert and 4 ml/well). Afterwards, 2 ml of a working solution consisting of fibronectin (5 μg/cm2), supplied by Chemicon, and PBS, supplied by GIBCO, was added and incubated over night at 4°C or 37°C. The next day, the solution was aspirated and membranes were allowed to dry for about 2 hrs before cells were added to inserts.

**Example 3**

Culturing of MSC on flat sheet membranes

(A) Seeding of cells

MSC were seeded under previously described conditions (see 1.2).
Figure 2 depicts the number of MSC which actually attached to the various surfaces. As can be seen, cells attached best to the membrane which was coated with FN/Hep/FGF-2. They also attached to the controls, TCPS and the membrane with fibronectin coating, however, fewer cells can be found on the control matrices.
As MSC are adherent cells, that means that they attach to the surface, seeding parameters were number of MSC per cm2 (MSC/cm2), which henceforth is referred to as seeding density) and media composition. Confluence of cells is reached when MSC cover the whole surface. In that case, cells must be detached from the surface and passaged to a new flask. For detachment cell culture media was removed, cells were rinsed with 8 ml PBS (phosphate-buffered saline). Afterwards, wash solution was removed and detachment enzyme was added. Then, cells were incubated with the detachment enzyme (incubation time respective to enzyme used) and later, cells were counted using a CASY counter, centrifuged at 300 g for 5 minutes and aliquots were transferred into a new medium. All volume specifications are given in respect to a surface of 75 cm2. Volumes for other surfaces can be calculated according to ratio.

(B) Trypsin

Trypsinization was performed by adding 3 ml Trypsin-EDTA after washing and a followed incubation time of 5 min in incubator. For cell detachment on inserts an incubation time of 10 min was selected, as detachment on the porous surface required a longer incubation time.

(C) Accutase

Detachment by Accutase was performed by adding 5 ml Accutase after rinsing with PBS and a followed incubation time of 30 min in incubator.

(D) Cell harvest

The process called cell harvest is principally the same as the one used for cell passage. The term cell harvest is used when any kind of cell analysis was performed after cell detachment. Cells were harvested after 7 days of proliferation. As can be seen in Figure 3, the number of MSC in 1000/cm$^2$ is highest for the membrane which was coated according to the invention with a FN/Hep/FGF-2 complex.

**Example 4**

Differentiation of cells

In order to assess the differentiation ability of cells which have been cultivated on membranes according to the invention, adipogenesis and osteogenesis differentiation tests were performed to determine the ability of MSC to differentiate.

Cells were seeded at 3000 MSCs/cm$^2$ in 6-well plates. Osteogenesis of MSCs was induced by addition of 1 ml standard medium supplemented with 10 mM β-glycerolphosphate, 50 μg/ml ascorbic acid and 100nM dexamethasone for a period of about two to three weeks. Media were exchanged three times a week. Subsequently cells were stained by van Kossa staining, which stains mineralised parts in the extracellular matrix. Cells were rinsed with 2 ml PBS/well, fixed with 2 ml 10% formaldehyde in PBS/well at minimum over night and washed twice with 2 ml double distilled water. 2 ml of 0.5% aqueous silver nitrate solution was added for 30 min at daylight. Cells were rinsed twice with 2 ml double distilled water prior to addition of a 0.5% aqueous sodium thiosulfate solution. Cells were repeatedly rinsed and counterstained with 0.05% aqueous Safranin-O-solution. Finally, cells were fixed in 2 ml 10% formaldehyde in PBS/well.

Cells were seeded at 21000 MSCs/cm$^2$ in 24-well plates and grown to post-confluence. Adipogenesis of MSCs was induced by addition of 2 ml standard medium supplemented with a hormonal cocktail consisting of 0.5 mM isobutyl-methylxanthine, 50 μg/ml ascorbic acid, 100 mM indomethacin, 100 μM dexamethasone, and 10 μg/ml insulin. Medium was exchanged three times a week for 11 days. Thereafter, standard medium supplemented with 10 μg/ml insulin was added to cells for 3 days. Cells were rinsed with 1 ml PBS/well and fixed with 1 ml 10% formaldehyde in PBS/well over night. 1 ml aqueous Red-oil-O staining solution (0.5 g Red-oil-O dissolved in 100 ml isopropanol and mixed 6:4 with double distilled) was added for 2 hrs at room temperature. Excess dye was removed with PBS (2 times washing with 1 ml PBS) and thereafter, cells were stored in 1 ml 10% formaldehyde in PBS.

Cells grown according to Example 3 differentiated into both adipogenic and osteogenic lineages indicating that stem cell potential was maintained. Exemplarily, brownish staining areas of mineralized matrix represent osteogenic differentiation, shown in Fig. 9A as dark spots, and red stained lipid droplets, shown in Fig. 9B as dark spots, prove that the cells underwent adipogenic differentiation.

**Claims**

1. A membrane which is modified on its surface with a complex comprising at least one extracellular matrix protein, one extracellular matrix (proteo-)glycan, wherein the glycan has the capacity to bind a growth factor, and a growth factor.

2. The membrane of claim 1, wherein the membrane is a polymer membrane comprising hydrophobic or hydrophilic polymers or both.

3. The membrane of claim 1 or 2, wherein the extracellular matrix protein is chosen from the group comprising fibronectin, collagen, pronectin F and laminin.

4. The membrane of any one of claims 1 to 3, wherein the extracellular matrix protein is fibronectin.

5. The membrane of any one of claims 1 to 4, wherein the extracellular matrix (proteo-)glycan is a glycosaminoglycan chosen from the group comprising heparin, heparin sulfate, hyaluronan, dermatan, keratan and chondroitin sulfate.

6. The membrane of claim 5, wherein the glycosaminoglycan is heparin or heparan sulfate.

7. The membrane of any one of claims 1 to 6, wherein the growth factor is chosen from the group comprising FGF-2, PDGF and EFG.

8. The membrane of any one of claims 1 to 7, wherein the growth factor is FGF-2.

9. The membrane of any one of claims 1 to 8, wherein said membrane is a flat sheet membrane or a hollow fiber membrane.

10. The membrane of any one of claims 1 to 9, wherein said membrane is modified on its surface with a complex comprising FGF-2, heparin and fibronectin.

11. The membrane of claim 10, wherein fibronectin is applied in a concentration of from $1\mu g/ml$ to $200\mu g/l$, FGF-2 is applied in a concentration of from 50 ng/ml to 500 ng/ml and heparin is applied in a concentration of from $1\mu g/ml$ to $200\mu g/ml$.

12. A process for preparing the membrane of any one of claims 1 to 11, comprising the steps of

   a) incubating a mixture comprising a growth factor, an extracellular matrix protein and an extracellular matrix (proteo-)glycan;
   b) exposing a polymer matrix to said mixture;
   c) removing the supernatant; and optionally
   d) rinsing the membrane.

13. The process of claim 11, wherein the mixture comprising a growth factor, an extracellular matrix protein and an extracellular matrix (proteo-)glycan is incubated together with the polymer matrix.

14. A process for cultivating cells, comprising the steps of

   (a) incubating the membrane of any of claims 1 to 11 with the cells at an appropriate temperature;
   (b) optionally exchanging the medium during the proliferation of the cells;
   (c) detaching the cells from the membrane;
   (d) optionally re-seeding the cells on a new membrane and repeating steps (a) to (c)or (a) to (d);
   (e) harvesting the cells;
   (f) optionally determining the cell count of the detached cells and/or characterizing the detached cells.

15. A membrane according to any of claims 1 to 11, wherein the membrane is populated with cells, preferably adherent cells.

16. A cell culturing device comprising a membrane according to any one of claims 1 to 11, or a membrane prepared according to claims 12 or 13.

17. A device for the extracorporeal treatment of body fluids, comprising cells and a membrane according to any one of claims 1 to 8, or a membrane prepared according to claim 9.

18. Use of a membrane according to any one of claims 1 to 11, or prepared according to claims 12 or 13, for the cultivation of cells.

19. Use according to claim 18, wherein the cells are renal cells.

20. Use according to claim 18, wherein the cells are mesenchymal stem cells.

heparin

fibronectin

Figure 1

Figure 2

Figure 3

Figure 4

Figure 5

Figure 6

Figure 7

Figure 8

Figure 9A

Figure 9B

Europäisches
Patentamt

European
Patent Office

Office européen
des brevets

# EUROPEAN SEARCH REPORT

Application Number

EP 10 17 0889

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/146261 A2 (UNIV CALIFORNIA [US]; HASHI CRAIG [US]; LI SONG [US]) 21 December 2007 (2007-12-21) | 1-3,5-9, 16,18-20 | INV. C12N5/00 |
| Y | * paragraphs [0058], [0124], [0145], [0293]; claims 1-40; figures 8, 9, 10, 12C,16, 32, 35 * | 1-20 | |
| Y | US 2004/161442 A1 (ZAMORA PAUL O [US] ET AL) 19 August 2004 (2004-08-19) * paragraphs [0012], [0 72], [0 90], [0 91], [ 100], [ 102] * | 1-20 | |
| Y | DEBLOIS C ET AL: "Heparin-fibroblast growth factorfibrin complex: in vitro and in vivo applications to collagen-based materials" BIOMATERIALS, ELSEVIER SCIENCE PUBLISHERS BV., BARKING, GB LNKD- DOI:10.1016/0142-9612(94)90164-3, vol. 15, no. 9, 1 January 1994 (1994-01-01), pages 665-672, XP024142424 ISSN: 0142-9612 [retrieved on 1994-01-01] * page 666, paragraph fibrin substrates.. * * page 666, paragraph collagen-based sponges * | 1-20 | |

-----

-----

-----

-/--

TECHNICAL FIELDS
SEARCHED (IPC)

C12N

The present search report has been drawn up for all claims

| Place of search | Date of completion of the search | Examiner |
|---|---|---|
| Munich | 24 September 2010 | Mabit, Hélène |

EPO FORM 1503 03.82 (P04C01)

Europäisches
Patentamt

European
Patent Office

Office européen
des brevets

# EUROPEAN SEARCH REPORT

Application Number

EP 10 17 0889

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZAMORA PAUL O ET AL: "Local delivery of basic fibroblast growth factor (bFGF) using adsorbed silyl-heparin, benzyl-bis(dimethylsilylmethyl)oxycarbamoyl-heparin" BIOCONJUGATE CHEMISTRY, vol. 13, no. 5, September 2002 (2002-09), pages 920-926, XP002601661 ISSN: 1043-1802 * page 924, paragraph effect of adhesion * * page 925 * | 1-20 | |
| A | BENOIT D S W ET AL: "Heparin functionalized PEG gels that modulate protein adsorption for hMSC adhesion and differentiation" ACTA BIOMATERIALIA, ELSEVIER, AMSTERDAM, NL LNKD- DOI:10.1016/J.ACTBIO.2005.03.002, vol. 1, no. 4, 1 July 2005 (2005-07-01), pages 461-470, XP025338364 ISSN: 1742-7061 [retrieved on 2005-07-01] | 1-20 | |
| A | MATSUBARA T ET AL: "A new technique to expand human mesenchymal stem cells using basement membrane extracellular matrix" BIOCHEMICAL AND BIOPHYSICAL RESEARCH COMMUNICATIONS, ACADEMIC PRESS INC. ORLANDO, FL, US LNKD- DOI:10.1016/J.BBRC.2003.11.143, vol. 313, no. 3, 16 January 2004 (2004-01-16), pages 503-508, XP004481585 ISSN: 0006-291X | 1-20 | TECHNICAL FIELDS SEARCHED (IPC) |

The present search report has been drawn up for all claims

| Place of search | Date of completion of the search | Examiner |
|---|---|---|
| Munich | 24 September 2010 | Mabit, Hélène |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 17 0889

This annex lists the patent family members relating to the patent documents cited in the above-mentioned European search report.
The members are as contained in the European Patent Office EDP file on
The European Patent Office is in no way liable for these particulars which are merely given for the purpose of information.

24-09-2010

| Patent document cited in search report | | Publication date | Patent family member(s) | Publication date |
|---|---|---|---|---|
| WO 2007146261 | A2 | 21-12-2007 | NONE | |
| US 2004161442 | A1 | 19-08-2004 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2007269489 A
- US 6150164 A
- US 6942879 A
- US 2006213836 A1
- US 2003138950 A1
- US 2007003916 A1
- US 6921811 B2
- US 20030203478 A1
- US 6942879 B
- US 4935141 A
- US 5891338 A
- EP 1578521 A1
- EP 2113298 A1
- EP 0305787 A1

### Non-patent literature cited in the description

- **ATALA.** Recent developments in tissue engineering and regenerative medicine. *Curr. Opin. Pediatr.,* 2006, vol. 16, 167-171
- **FISSELL.** *Expert Rev. Med. Devices,* 2006, vol. 3 (2), 155
- **ISHIHARA et al.** Heparin-carrying polystyrene to mediate cellular attachment and growth via interaction with growth factors. *Journal of Biomedical Materials Research,* 2000, vol. 50 (2), 144-152
- **ATALA A et al.** Tissue-engineered autolo-gous bladders for patients needing cystoplasty. *Lancet,* 2006, vol. 367, 1241-6